# EUROPÄISCHE PATENTSCHRIFT

(11) **EP 1 320 319 B1**
(45) Veröffentlichungstag und Bekanntmachung des Hinweises auf die Patenterteilung: **17.12.2003**
(21) Anmeldenummer: 02748829.5
(22) Anmeldetag: 28.06.2002
(51) Int. Cl.: A61B 1/00, G02B 23/24

(54) **VORRICHTUNG ZUM POSITIONIEREN ZUMINDEST EINES BAUELEMENTS INNERHALB EINES ENDOSKOPISCHEN SYSTEMS**
DEVICE FOR POSITIONING AT LEAST ONE COMPONENT IN AN ENDOSCOPIC SYSTEM
DISPOSITIF POUR POSITIONNER AU MOINS UN COMPOSANT A L'INTERIEUR D'UN SYSTEME ENDOSCOPIQUE

(30) Priorität: 24.07.2001 DE 10136998
(43) Veröffentlichungstag der Anmeldung: 25.06.2003
(73) Patentinhaber: Karl Storz GmbH & Co. KG, 78532 Tuttlingen (DE)
(72) Erfinder: KEHR, Ulrich, 70771 Leinfelden-Echterdingen (DE)
(74) Vertreter: Heuckeroth, Volker, Dr.
(86) Internationale Anmeldenummer: EP0207140
(87) Internationale Veröffentlichungsnummer: WO03011120

(56) Entgegenhaltungen:
- WO-A-00/45210
- DE-A- 4 211 203
- DE-A- 19 718 189
- DE-U- 8 810 044
- PATENT ABSTRACTS OF JAPAN vol. 008, no. 058 (P-261), 16. März 1984 (1984-03-16) & JP 58 208721 A (FUJI SHASHIN KOKI KK), 5. Dezember 1983 (1983-12-05)

## Beschreibung

Die Erfindung betrifft eine Vorrichtung zum Positionieren zumindest eines Bauelements innerhalb eines endoskopischen Systems, mit einem hermetisch dichten Gehäuse, mit zumindest einem äußeren magnetisch wirksamen Element, das außerhalb des Gehäuses angeordnet ist, und mit zumindest einem inneren magnetisch wirksamen Element, das innerhalb des Gehäuses angeordnet ist, wobei zwischen dem äußeren Element und dem inneren Element durch das Gehäuse hindurch ein magnetischer Kraftschluß wirkt, wobei ferner das äußere Element und das innere Element bzgl. einer Längsachse des Gehäuses zumindest mit axialer Bewegungskomponente beweglich sind und das innere Element mit dem Bauelement derart in Wirkverbindung steht, daß eine Bewegung des inneren Elements eine Bewegung des Bauelements bewirkt.

Eine derartige Vorrichtung ist aus dem deutschen Gebrauchsmuster DE 88 10 044 U1 bekannt.

Im Sinne der vorliegenden Erfindung umfaßt der Begriff Bauelement beispielsweise optische Bauelemente, z.B. Linsen mit oder ohne Fassung in einem Optikkopf eines Endoskopes. Ein derartiges optisches Bauelement kann beispielsweise Teil einer Fokussiereinrichtung eines Endoskops zum Fokussieren oder zum Scharfstellen des endoskopischen Bildes sein. Unter den Begriff Bauelemente fallen jedoch auch mechanische Bauelemente.

Unter endoskopischen Systemen im Sinne der vorliegenden Erfindung werden Endoskope oder auch endoskopische Kamerasysteme verstanden.

Unter einem hermetisch dichten Gehäuse im Sinne der vorliegenden Erfindung versteht man ein derart abgeschlossenes Gehäuse, das beispielsweise autoklaviert werden kann, ohne daß die Gefahr besteht, daß bei den extremen Temperaturschwankungen in den Innenraum des Gehäuses Feuchtigkeit oder Flüssigkeiten, somit Kontaminationen eindringen können.

Unter einem magnetisch wirksamen Element im Sinne der vorliegenden Erfindung wird ein Magnet, beispielsweise ein Permanentmagnet, oder auch ein Element oder Material verstanden, das unter dem Einfluß eines Magnetfeldes magnetisierbar ist, oder auch ein Elektromagnet. So kann beispielsweise das zumindest eine äußere magnetisch wirksame Element ein Permanentmagnet, und das zumindest eine innere magnetisch wirksame Element ein magnetisierbares ferromagnetisches Material oder Element sein, oder umgekehrt. Es ist ausreichend, wenn das zumindest eine äußere magnetisch wirksame Element oder das zumindest eine innere magnetisch wirksame Element ein Magnetfeld erzeugt, und das jeweils andere Element dann unter dem Einfluß dieses Magnetfeldes mit dem das Magnetfeld erzeugenden Element magnetisch kraftschlüssig zusammenwirkt. Der magnetische Kraftschluß zwischen dem zumindest einen äußeren magnetisch wirksamen Element und dem zumindest einen inneren magnetisch wirksamen Element wirkt dabei durch das Gehäuse durch, wodurch eine sogenannte Magnetkupplung realisiert wird.

Bei der aus dem eingangs genannten Gebrauchsmuster bekannten Vorrichtung ist das zumindest eine äußere magnetisch wirksame Element an der Innenseite eines konzentrisch um die Gehäuseachse angeordneten äußeren Ringes angeordnet, und das zumindest eine innere magnetisch wirksame Element ist in Form eines Ringmagneten innerhalb des Gehäuses in einer Gleitführung geführt und steht mit dem zu positionierenden Bauelement fest in Verbindung. Der äußere Ring, der das äußere magnetisch wirksame Element trägt, dient als Betätigungselement, und durch Drehen des Ringes wird der Ring und damit das äußere magnetisch wirksame Element entlang einer Schraubenführung axial bewegt, wodurch das innere magnetisch wirksame Element ebenfalls axial bewegt wird. Bei dieser bekannten Vorrichtung erstrecken sich das äußere magnetisch wirksame Element und das innere magnetisch wirksame Element vollumfänglich um die Längsachse des Gehäuses. Eine derartige vollumfängliche Anordnung dieser Elemente kann die radiale Anziehungskraft zwischen dem inneren und äußeren Element derart kompensieren, daß das innere Element nicht gegen einen reibungsbehafteten Gleitsitz gedrückt wird, was bei der axialen Bewegung des inneren Elements einen erhöhten Reibschluß bewirken würde, wodurch die axiale Bewegung des inneren Elements und damit des zu positionierenden Bauelements nicht ruckfrei erfolgen könnte. Die vollumfängliche Anordnung des äußeren und inneren Elements bedingt jedoch einen erhöhten Kostenaufwand der Vorrichtung. Würden sich das äußere magnetisch wirksame Element und das innere magnetisch wirksame Element nur über einen Teilumfang des Gehäuses der Vorrichtung erstrecken, bestünde dagegen der Nachteil, daß die radiale Anziehungskraft zwischen den Elementen nicht kompensiert würde, was bei einer Gleitführung des inneren und/oder äußeren Elements zu einer erhöhten Reibung bei deren Bewegung führen würde, wodurch eine ruckfreie Positionierung des zumindest einen Bauelements nicht gewährleistet werden könnte.

Eine andere Bauweise einer Vorrichtung zum Positionieren von Bauelementen innerhalb endoskopischer Systeme ist aus der DE 197 13 276 A1 bekannt, bei der mehrere äußere magnetisch wirksame Elemente an einem außerhalb des Gehäuses konzentrisch zur Gehäuseachse angeordneten Ringelement und mehrere innere magnetisch wirksame Elemente an einem innerhalb des Gehäuses ebenfalls konzentrisch zur Gehäuseachse angeordneten Ringelement angeordnet sind. Sowohl das äußere Ringelement als auch das innere Ringelement sind drehbar, jedoch axial unverschieblich. Um durch Drehen des äußeren Ringelements eine axiale Bewegung des zumindest einen zu positionierenden Bauelements zu erzielen, steht der innere Ring über eine Art Getriebe in Form einer Wendelnut mit dem Bauelement mechanisch in Verbindung, um die Drehung des äußeren Ringes in eine translatorische Bewegung des Bauelements zu übertragen. Die Steigung der Wendelnut bestimmt dabei das Verhältnis zwischen dem Drehwinkel des äußeren Ringelements und dem Verschiebeweg des zu positionierenden Bauelements. Die Steigung kann jedoch nicht beliebig gewählt werden, insbesondere ist sie nach oben begrenzt, da sonst eine Selbsthemmung des Getriebes auftritt. Besonders bei großen Steigungen der Wendelnut kann sich die Reibung störend in einer ruckhaften Bewegung auswirken. In der Praxis muß daher die Steigung so gewählt werden, daß das äußere Ringelement immer um einen relativ großen Winkel verdreht werden muß, um eine vergleichsweise kleine translatorische Bewegung zu erhalten. Bei Vorrichtungen, beispielsweise Fokussiereinrichtungen, in denen große Verstellwege schnell bewältigt werden sollen, ist dies jedoch nachteilig.

Aus der US 5,359,992 ist eine Vorrichtung zum Positionieren von Bauelementen innerhalb endoskopischer Systeme bekannt, bei der in einem äußeren Ringelement, das wiederum konzentrisch zur Gehäuseachse um das Gehäuse herum angeordnet ist, diametral gegenüberliegend zwei schraubenlinienförmige Schlitze ausgebildet sind, in denen jeweils diametral gegenüberliegend Rundmagnete eingesetzt sind. Die Rundmagnete greifen in eine axial verlaufende Aussparung an der Außenseite einer in dem Ring angeordneter Hülse ein. Ein Drehen des äußeren Ringes verursacht somit eine Axialverschiebung des äußeren Magneten. Im inneren abgeschlossenen Bereich sind entsprechend diametral gegenüberliegende Magnete vorhanden, die den Bewegungen des äußeren Magneten Folgen und somit die Kupplung bewirken. An diese Ausgestaltung ist die stark reibungsbehaftete flächige Gleitführung der inneren Magnete und hier sogar auch der äußeren Magnete nachteilig.

Das gleiche Problem besteht bei der aus der US 5,056,902 bekannten Vorrichtung zum Positionieren von Bauelementen innerhalb endoskopischer Systeme, die dort als Fokussiereinrichtung dient. Bei den vorgenannten bekannten Vorrichtungen, bei denen sowohl das äußere magnetisch wirksame Element als auch das innere magnetisch wirksame Element zumindest mit axialer Bewegungskomponente verschiebbar sind, bestehen die Nachteile in einer reibungsbehafteten Gleitführung mit flächiger Auflage des zumindest einen inneren magnetisch wirksamen Elements, die aufgrund der Anziehung zwischen dem äußeren und dem inneren Element erheblich reibungsbehaftet ist. Durch die reibungsbehaftete Gleitführung des zumindest einen inneren magnetisch wirksamen Elements innerhalb des Gehäuses kommt es jedoch mitunter zu ruckartigen Bewegungen des zumindest einen Bauelements bei dessen Positionierung, da zu Beginn jeder axialen Bewegung des zumindest einen äußeren magnetisch wirksamen Elements zunächst die nicht geringe Haftreibung des inneren magnetisch wirksamen Elements überwunden werden muß. Dadurch ist jedoch eine exakte Positionierung des zumindest einen Bauelements nicht immer gewährleistet.

Die Minderung der Reibung bei der Bewegung der magnetisch wirksamen Elemente kann bei den bekannten Vorrichtungen nur durch eine vollumfängliche Ausgestaltung der Elemente oder durch zumindest eine rotationssymmetrische Anordnung der Elemente erzielt werden, was wiederum mit einem erhöhten Konstruktionsaufwand verbunden ist.

Der Erfindung liegt daher die Aufgabe zugrunde, eine Vorrichtung der eingangs genannten Art dahingehend weiterzubilden, daß die Magnetkupplung bei wenig aufwendiger Bauweise eine möglichst ruckfreie Positionierung des zumindest einen Bauelements ermöglicht.

Erfindungsgemäß wird diese Aufgabe hinsichtlich der eingangs genannten Vorrichtung dadurch gelöst, daß das zumindest eine innere Element in Bezug auf die Richtung der Anziehungskraft des äußeren Elements über eine Halterung in dem Gehäuse zumindest mit axialer Bewegungskomponente beweglich hängend angeordnet ist, wobei eine dem äußeren Element zugewandte Seite des inneren Elements frei ist, und daß das Bauelement über ein Mitnehmerelement mit dem inneren Element derart in Wirkverbindung steht, daß das Bauelement bei einer axialen Bewegung des inneren Elements axial verschoben wird.

Die erfindungsgemäße Vorrichtung beruht somit auf dem Prinzip, das zumindest eine innere magnetisch wirksame Element in Bezug auf die Richtung der Anziehungskraft des äußeren Elements axial beweglich aufzuhängen, anstatt das innere Element in Gleitführungen wie Nuten oder dergleichen anzuordnen. Die Halterung nimmt somit die in Richtung auf das äußere magnetisch wirksame Element wirkende Anziehungskraft auf, während das innere Element auf seiner dem äußeren Element zugewandten Seite selbst frei ist, mit anderen Worten in dem Gehäuse "schwebend" oder "fliegend" angeordnet ist. Das innere Element unterliegt somit bei seiner axialen Bewegung keinen Reibungskräften wie in einer flächigen Gleitführung, auf die hier verzichtet werden kann. Auch das zumindest eine zu positionierende Bauelement, das lediglich über ein Mitnehmerelement mit dem inneren Element in Wirkverbindung steht, wird somit nicht mit der Anziehungskraft des äußeren Elements beaufschlagt, wodurch auch das Bauelement besonders reibungsarm innerhalb des Gehäuses verschoben werden kann, wenn sich das innere Element axial bewegt. Durch die Aufhängung des inneren Elements ist es darüber hinaus besonders vorteilhaft möglich, eine bezüglich der Gehäuseachse asymmetrische Anordnung des inneren und des äußeren magnetisch wirksamen Elements vorzusehen, da keine Kompensensation der radialen Anziehungskraft erforderlich ist, wodurch der Teile- und damit Kostenaufwand der erfindungsgemäßen Vorrichtung verringert werden kann. Des weiteren ermöglicht es die erfindungsgemäße Bauweise der Vorrichtung anstelle eines im Stand der Technik bekannten konzentrisch zur Gehäuseachse angeordneten äußeren Ringelements ein seitliches Stellrad zur Betätigung der Magnetkupplung vorzusehen, wodurch große Verstellwege des Bauelements schnell bewältigt werden können. Die Aufhängung des zumindest einen inneren Elements kann beispielsweise in Form von Gleitrollen oder dergleichen oder durch eine gelenkig aufgehängte Halterung in der Art eines Pendels bewerkstelligt werden.

Besonders bevorzugt ist es, wenn das innere Element mittels der Halterung um zumindest eine Schwenkachse pendelnd in dem Gehäuse aufgehängt ist.

Der Vorteil dieser Maßnahme besteht zum einen darin, daß bei einer pendelnden Aufhängung des zumindest einen inneren Elements um zumindest eine Schwenkachse eine besonders reibungsarme axiale Beweglichkeit des inneren Elements erreicht wird, bei der die Anziehungskräfte der magnetisch wirksamen Elemente allein von der zumindest einen Schwenkachse aufgenommen werden, und daß zum anderen eine konstruktiv einfache und damit sehr kostengünstige Bauweise erzielt wird.

In einer weiteren bevorzugten Ausgestaltung ist das Bauelement in einer Führung axial linear geführt.

Aufgrund der erfindungsgemäßen Ausgestaltung der Magnetkupplung, nach der das zumindest eine innere Element in dem Gehäuse frei beweglich aufgehängt ist und das Bauelement aufgrund der Wirkverbindung mit dem inneren Element über das Mitnehmerelement keinerlei Radialkräfte aufnehmen muß, läßt sich durch diese Maßnahme eine besonders reibungsgünstige Führung des Bauelements innerhalb des Gehäuses realisieren. Auf das Bauelement wirken aufgrund des Mitnehmerelements lediglich axiale Kräfte, wodurch eine besonders reibungsarme Führung des Bauelements erreicht wird.

In einer weiteren bevorzugten Ausgestaltung ist die Halterung mit einem ersten Ende über zumindest ein Gelenk im Gehäuse aufgehängt und trägt am gegenüberliegenden Ende das innere Element.

Diese Maßnahme stellt eine konstruktiv besonders einfache Ausgestaltung für die gemäß einer zuvor genannten Ausgestaltung pendelartige Aufhängung des inneren Elements dar. Die Halterung kann sich dabei vorteilhafterweise diametral durch den Innenquerschnitt des Gehäuses erstrecken, wodurch im wesentlichen der gesamte Innendurchmesser des Gehäuses zur Verfügung steht, so daß auch bei kleinen Auslenkungen der Halterung um die zumindest eine Schwenkachse ein großer Bewegungsweg des inneren Elements erzielt werden kann.

Dabei ist es bevorzugt, wenn das innere Element gelenkig mit der Halterung verbunden ist, derart, daß sich die Winkellage des inneren Elements in Bezug auf die Längsachse des Gehäuses bei der Pendelbewegung nicht ändert.

Diese Maßnahme ist insbesondere bei sehr großen Verstellwegen und damit großen Auslenkungen der Halterung aus ihrer Ruhelage von Vorteil, da sich durch diese Maßnahme nicht die Orientierung zwischen dem inneren Element und dem äußeren Element verändert und somit im wesentlichen der magnetische Kraftschluß zwischen diesen beiden Elementen auch bei großen Verstellwegen und großen Auslenkungen der Halterung nicht verlorengeht.

In einer weiteren bevorzugten Ausgestaltung verläuft die Halterung seitlich um das Bauelement.

Durch diese Maßnahme wird der Vorteil einer besonders platzsparenden und wenig raumergreifenden Ausgestaltung der erfindungsgemäßen Vorrichtung erreicht, wobei sich bei dieser Ausgestaltung die Halterung im wesentlichen über den gesamten Innendurchmesser des Gehäuses erstrecken kann. Diese Anordnung ist insbesondere dann vorteilhaft, wenn die Handhabung der Positioniervorrichtung und das Bauelement axial nahe beieinander oder gar auf gleicher Höhe angeordnet sind.

In einer weiteren bevorzugten Ausgestaltung steht die Halterung mit dem Bauelement über das Mitnehmerelement mit radialem Spiel bezüglich der Längsrichtung des Gehäuses in Wirkverbindung.

Diese Maßnahme ist im Rahmen der pendelartigen Ausgestaltung der Halterung von Vorteil, da bei einer solchen pendelartigen Ausgestaltung der Halterung diese nicht nur eine Bewegungskomponente in Längsrichtung der Gehäuseachse, d.h. axial, besitzt, sondern entsprechend der Verschwenkung um die zumindest eine Schwenkachse auch eine radiale Bewegungskomponente. Die zuvor genannte Maßnahme bewirkt nun vorteilhafterweise, daß sich aufgrund des radialen Spiels zwischen der Halterung und dem Bauelement über die Verbindung durch das Mitnehmerelement durch die radiale Bewegungskomponente der Halterung keine radialen Zwangskräfte auf das Bauelement übertragen, so daß dieses weiterhin insbesondere in der linearen Führung reibungsarm verschoben werden kann.

In einer weiteren bevorzugten Ausgestaltung ist das Bauelement von der Halterung axial beabstandet, und ist das Mitnehmerelement als längliches Zug- und Schubelement ausgebildet, das mit der Halterung einerseits und mit dem Bauelement andererseits, vorzugsweise jeweils gelenkig, verbunden ist.

Diese Maßnahme ist dann von Vorteil, wenn das zu positionierende Bauelement im distalen Bereich angeordnet ist, die Handhabung der Positioniervorrichtung jedoch am proximalen Ende des endoskopischen Systems, beispielsweise aus ergonomischen Gründen, erfolgen soll. Auch in diesem Fall wird durch die erfindungsgemäße Ausgestaltung ein reibungsarmes und damit ruckfreies Positionieren des zumindest einen Bauelements erzielt.

In einer weiteren bevorzugten Ausgestaltung ist das äußere Element mit einem Bedienungselement verbunden, das seitlich am Gehäuse angeordnet ist.

Diese Maßnahme, die vor allem durch die erfindungsgemäße Ausgestaltung der Magnetkupplung der vorliegenden Vorrichtung ermöglicht wird, hat den Vorteil einer ergonomisch besonders günstigen Handhabung der Vorrichtung, während bei den aus dem Stand der Technik bekannten Vorrichtungen das Bedienungselement stets in Form eines konzentrisch zur Gehäuseachse um das Gehäuse herum angeordneten Stellrings ausgebildet ist. Der Vorteil eines seitlich am Gehäuse angeordneten Bedienungselements besteht darin, daß es eine Einhandbedienung ermöglicht, d.h. die Vorrichtung kann beispielsweise mit dem Daumen derselben Hand, die das endoskopische System hält, zum Positionieren des zumindest einen Bauelements bedient werden. Das Bedienungselement kann beispielsweise in Form eines Schiebers ausgebildet sein.

Bevorzugter ist es jedoch, wenn das Bedienungselement als Stellrad mit etwa quer zur Längsachse verlaufender Drehachse ausgebildet ist, das mit dem äußeren Element in Wirkverbindung steht, wobei eine Drehbewegung des Stellrades eine Bewegung des äußeren Elements mit axialer Bewegungskomponente bewirkt.

Eine solche Ausgestaltung des Bedienungselements hat den Vorteil einer besonders ergonomisch günstigen Handhabung der erfindungsgemäßen Vorrichtung, indem das Stellrad mit dem Daumen betätigt werden kann. Insbesondere in Verbindung mit der erfindungsgemäßen Magnetkupplung der vorliegenden Vorrichtung läßt sich ein solches ergonomisch günstiges Bedienungselement bei einem aufgrund der erfindungsgemäßen Magnetkupplung autoklavierbaren endoskopischen System realisieren.

Weitere Vorteile und Merkmale ergeben sich aus der nachfolgenden Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung, wie in den angefügten Ansprüchen definiert, zu verlassen.

Ausführungsbeispiele der Erfindung sind in der Zeichnung dargestellt und werden mit Bezug auf diese hiernach näher beschrieben. Es zeigen:
- Fig. 1: ein endoskopisches System in einer perspektivischen Gesamtdarstellung, bei dem eine erfindungsgemäße Vorrichtung zum Positionieren zumindest einen Bauelements vorhanden ist;
- Fig. 2: einen gegenüber Fig. 1 vergrößerten Längsschnitt durch das proximale Ende des endoskopischen Systems in Fig. 1, in dem die Positioniervorrichtung angeordnet ist;
- Fig. 3: das Funktionsprinzip der Positioniervorrichtung gemäß Fig. 2 in einer vereinfachten schematischen Längsschnittdarstellung in einem ersten Betriebszustand;
- Fig. 4: eine Fig. 3 vergleichbare schematische Darstellung in einer zweiten Betriebsstellung der Positioniervorrichtung; und
- Fig. 5: ein weiteres Ausführungsbeispiel einer Positioniervorrichtung in einer ebenfalls schematischen Darstellung.

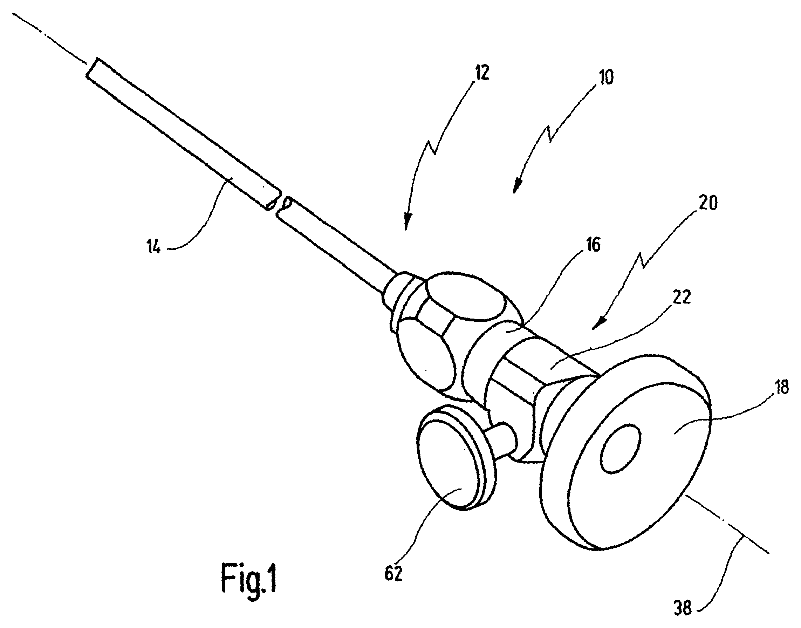

In Fig. 1 ist ein mit dem allgemeinen Bezugszeichen 10 versehenes endoskopisches System dargestellt. Das endoskopische System 10 ist in dem gezeigten Ausführungsbeispiel ein Endoskop 12.

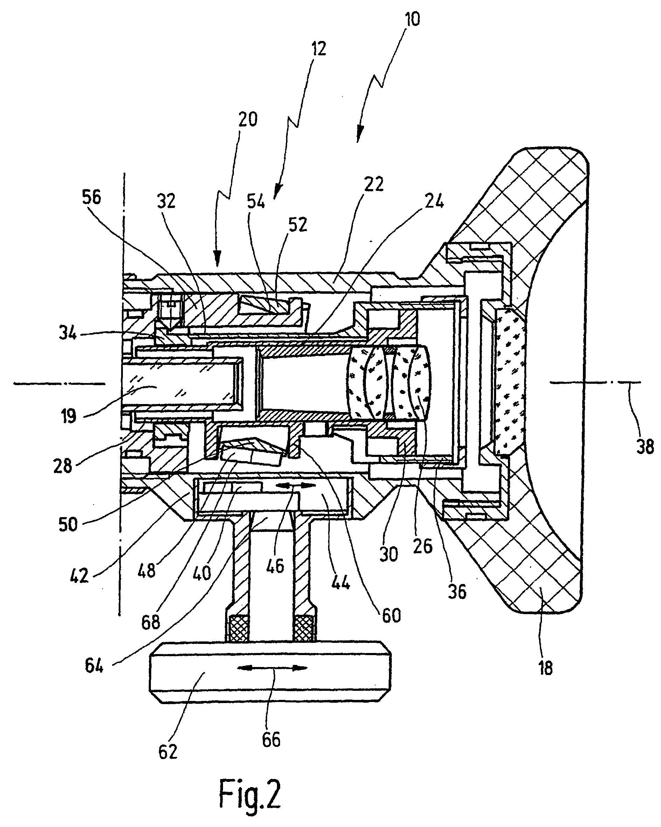

Das Endoskop 12 weist einen Schaft 14 auf, der proximalseitig mit einem Optikkopf 16 verbunden ist, an dessen proximalem Ende ein Okular bzw. eine Okularmuschel 18 angeordnet ist. In dem Schaft 14 und durch den Optikkopf 16 hindurch verläuft ein (nicht näher dargestelltes) Bildübertragungssystem, das als Stablinsensystem ausgebildet ist, dessen proximales Ende in Fig. 2 mit dem Bezugszeichen 19 versehen ist.

In dem Endoskop 12 ist ferner eine Vorrichtung 20 zum Positioneren zumindest eines Bauelements innerhalb des Endoskops 12 vorhanden, die mit Bezug auf Fig. 1 und Fig. 2 nachfolgend näher beschrieben wird.

Die Vorrichtung 20 weist ein hermetisch dichtes Gehäuse 22 auf, das im wesentlichen hohlzylindrisch ausgebildet ist. Das Gehäuse 22 ist Teil des Optikkopfes 16. Während das Gehäuse 22 in Umfangsrichtung voll umfänglich durchgehend ist, d.h. keine Öffnungen oder Durchbrechungen aufweist, ist das Gehäuse 22 auf Seiten des Okulars hermetisch dicht verschlossen, und auch auf Seiten des Schafts 14 dicht verschlossen, so daß die Vorrichtung 20 hermetisch dicht und damit autoklavierbar ist.

Innerhalb des Gehäuses 22 ist ein zu positionierendes Bauelement 24 angeordnet. Das Bauelement 24 ist hier als Fassung für eine Mehrzahl von Linsen 26 ausgebildet, die durch entsprechende axiale Lageverstellung, wie hiernach noch näher beschrieben wird, dem Fokussieren und/oder Scharfstellen des von dem Bildübertragungssystem übertragenen endoskopischen Bildes dienen.

Das Bauelement 24 ist insgesamt hülsenartig aufgebaut und weist ein distales Ende 28 sowie ein proximales Ende 30 auf.

Das Bauelement 24 ist axial linear in einer Führung 32 geführt, die als Hülse ausgebildet ist und einen distalen Führungsabschnitt 34, in dem das distale Ende 28 des Bauelements 24 axial linear geführt ist, und einen proximalen Führungsabschnitt 36 aufweist, in dem das proximale Ende 30 des Bauelements 24 axial linear geführt ist.

Innerhalb der Führung 32 ist das Bauelement 24 somit axial linear geführt und gegebenenfalls gegen ein Verdrehen um eine Längsachse 38 gesichert.

Die Längsachse 38 bildet die Längsachse des Schafts 14 und auch die Längsachse des Gehäuses 22 und der Vorrichtung 20.

Die Vorrichtung 20 weist weiterhin zumindest ein äußeres magnetisch wirksames Element 40, beispielsweise einen Permanentmagneten auf, der außerhalb des Gehäuses 22 angeordnet ist. Das äußere magnetisch wirksame Element 40 ist im vorliegenden Ausführungsbeispiel in einen verdickten Abschnitt 42 des Gehäuses 22 integriert, worunter ebenfalls verstanden wird, daß das Element 40 außerhalb des Gehäuses 22 angeordnet ist. In dem verdickten Abschnitt 42 ist eine entsprechende Aussparung 44 vorhanden, in der das Element 40 angeordnet ist. Wie aus Fig. 2 hervorgeht, trennt das Gehäuse 22 auch im Bereich der Aussparung 44 den Außenraum des Gehäuses 22 hermetisch dicht von dem Innenraum des Gehäuses 22.

Das äußere magnetisch wirksame Element 40 ist zumindest mit axialer Bewegungskomponente gemäß einem Doppelpfeil 46 beweglich.

Die Vorrichtung 20 weist ferner zumindest ein innerhalb des Gehäuses 22 angeordnetes inneres magnetisch wirksames Element 48 auf, das dem äußeren Element 40 gegenüberliegend angeordnet ist.

Das äußere Element 40 und das innere Element 48 erstrecken sich nur über einen Teilumfang des Gehäuses 22.

Das innere magnetisch wirksame Element 48, beispielsweise ebenfalls ein Permanentmagnet oder einfach ein magnetisierbares Material, beispielsweise ein ferromagnetisches Material, steht mit dem äußeren Element 40 entsprechend über einen magnetischen Kraftschluß wirkend in Verbindung, wobei der magnetische Kraftschluß durch das hermetisch dicht geschlossene Gehäuse 22 hindurch wirkt, das entsprechend das Magnetfeld nicht abschirmt. Der magnetische Kraftschluß besteht darin, daß sich das innere und das äußere Element 40, 48 gegenseitig anziehen.

Auch das innere magnetisch wirksame Element 48 ist zumindest mit axialer Bewegungskomponente beweglich, wie hiernach noch näher beschrieben wird.

Das innere Element 48 ist an einer Halterung 50 angeordnet bzw. befestigt, wobei das innere magnetisch wirksame Element 48 auch in einem beispielsweise magnetisierbaren Abschnitt der Halterung 50 bestehen kann.

Über die Halterung 50 ist das innere magnetisch wirksame Element 48 in dem Gehäuse 22 in Bezug auf die Richtung der Anziehungskraft des äußeren Elements 40, die im wesentlichen quer zur Längsachse 38 bzw. radial bezüglich dieser durch das Gehäuse 22 hindurch verläuft, in dem Gehäuse hängend angeordnet.

Die Halterung 50 erstreckt sich im wesentlichen quer zur Längsachse 38 des Gehäuses 22 und ist mit einem Ende 52, das dem jeweiligen Ende der Halterung 50 gegenüberliegt, an dem das innere magnetisch wirksame Element 48 angeordnet ist, über zumindest ein Gelenk 54 am Gehäuse 22 befestigt, und zwar im gezeigten Ausführungsbeispiel indirekt über ein Lagerteil 56, das seinerseits fest mit dem Gehäuse 22 verbunden ist.

Das Gelenk 54 besteht in dem gezeigten Ausführungsbeispiel einfach darin, daß das Ende 52 der Halterung 50 in das Lagerteil 56 eingehängt ist und durch eine entsprechende Formgebung des Endes 52 in dem Lagerteil 56 einen Auflagepunkt bildet, um den die Halterung 50 pendeln bzw. verschwenken kann. Das Gelenk 54 bildet entsprechend eine Schwenkachse aus, um die die Halterung 50 pendelnd in dem Gehäuse 22 aufgehängt ist.

Die Halterung 50 ist im vorliegenden Ausführungsbeispiel in Form eines Ringes ausgebildet, wobei das Bauelement 24 durch die Halterung 50 hindurchgeht.

Wie weiterhin aus Fig. 2 hervorgeht, erstreckt sich die Halterung 50 bezüglich der mechanischen Wirkungslinie zwischen ihrer Aufhängung an dem Ende 52 und dem gegenüberliegenden Ende, an dem das innere Element 48 angeordnet ist, im wesentlichen diametral durch den Innenraum des Gehäuses 22 und parallel zur Wirkungsrichtung der durch das äußere Element 40 auf das innere Element 48 ausgeübten Anziehungskraft. Genauer gesagt fluchtet diese Wirkungslinie sogar-mit der Anziehungskraft in der nicht ausgeschwenkten Position der Halterung 50, wodurch das Gelenk 54 die Anziehungskraft optimal aufnehmen kann.

Das Bauelement 24 steht mit dem inneren magnetisch wirksamen Element 48 über ein Mitnehmerelement 60 in Wirkverbindung, wobei das Mitnehmerelement 60 in Form zweier radial von dem Bauelement 24 abstehenden Vorsprüngen ausgebildet ist, die an der Halterung 50 angreifen.

Bei einer Auslenkung der Halterung 50 um die durch das Gelenk 54 gebildete Schwenkachse wird das Bauelement 24 entsprechend in der Führung 32 linear in Richtung der Längsachse 38 verschoben.

Die Halterung 50 und damit das innere Element 48 ist mit dem Bauelement 24 dabei über das Mitnehmerelement 60 mit radialem Spiel bezüglich der Längsachse 38 des Gehäuses 22 verbunden, was in dem gezeigten Ausführungsbeispiel durch die bloße Anlage des Mitnehmerelements 60 in Form der beiden radial abstehenden Vorsprünge an der Halterung 50 erreicht wird.

Es versteht sich, daß das mit dem Bezugszeichen 54 versehene Gelenk nicht nur so zu verstehen ist, daß ein solches Gelenk eine starre Schwenkachse definiert, sondern es könnte auch eine Aufhängung der Halterung 50 über ein flexibles Element in dem Gehäuse 22 realisiert sein, worunter ebenfalls eine gelenkige Verbindung mit dem Gehäuse 22 verstanden wird, die eine Schwenkachse definiert, wobei eine solche Schwenkachse räumlich nicht scharf definiert sein muß. Auch könnte die Halterung 50 mehrere Aufhängungspunkte aufweisen, die durch mehrere Gelenke gebildet werden.

Wie aus Fig. 2 hervorgeht, bilden das äußere magnetische wirksame Element 40 und das innere magnetisch wirksame Element 48 bezüglich der Längsachse 38 des Gehäuses 22 eine asymmetrische Anordnung, weil sie sich nur über einen Teilumfang des Gehäuses 22 erstrecken, was den Vorteil hat, daß, wie in Fig. 2 dargestellt, zur axialen Bewegung des äußeren Elements 40 ein seitlich am Gehäuse 22 angeordnetes Bedienungselement 62 vorgesehen werden kann. Das Bedienungselement 62 ist in Form eines Stellrades ausgebildet, dessen Drehachse quer zur Längsachse 38 verläuft. Das Stellrad ist über einen Zapfen 64 mit dem äußeren Element 40 fest verbunden. Eine Drehbewegung des Stellrades in eine Richtung gemäß einem Doppelpfeil 66 bewirkt dabei eine Schwenkbewegung des äußeren Elements 40 um die Drehachse des Stellrades mit einer axialen Bewegungskomponente in der entsprechenden Richtung des Doppelpfeiles 46.

Die umfängliche Erstreckung des inneren Elements 48 ist entsprechend an den Schwenkradius des äußeren Elements 40 angepaßt, um stets einen magnetischen Kraftschluß zwischen beiden Elementen 40, 48 zu gewährleisten.

Das Stellrad könnte auch über ein Getriebe mit dem äußeren Element 40 verbunden sein, das eine Drehbewegung des Stellrades in eine ausschließlich axial lineare Bewegung des äußeren Elements 40 umsetzt.

Wie weiterhin aus Fig. 2 hervorgeht, ist das innere magnetisch wirksame Element 48 in dem Gehäuse 22 derart aufgehängt, daß seine dem äußeren Element 40 zugewandte Seite 68 frei ist, d.h. keiner Reibung unterliegt.

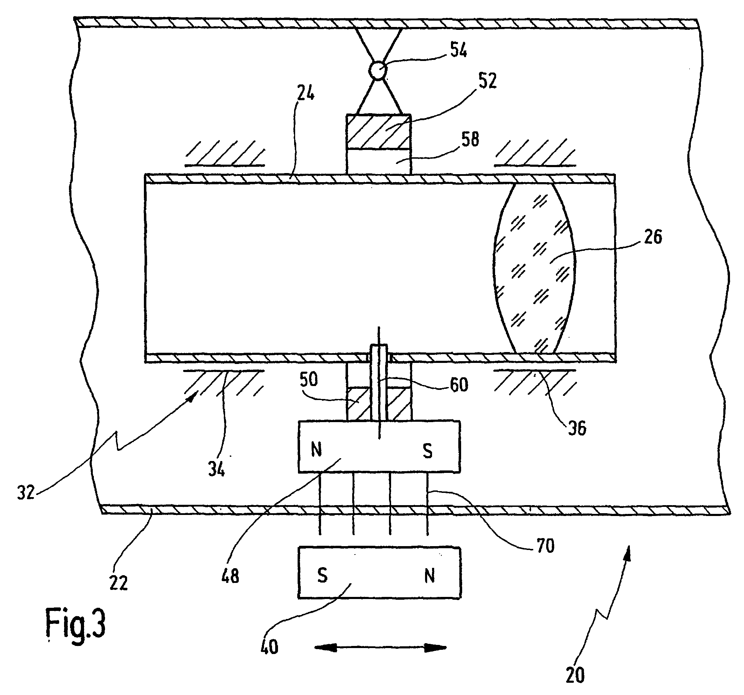

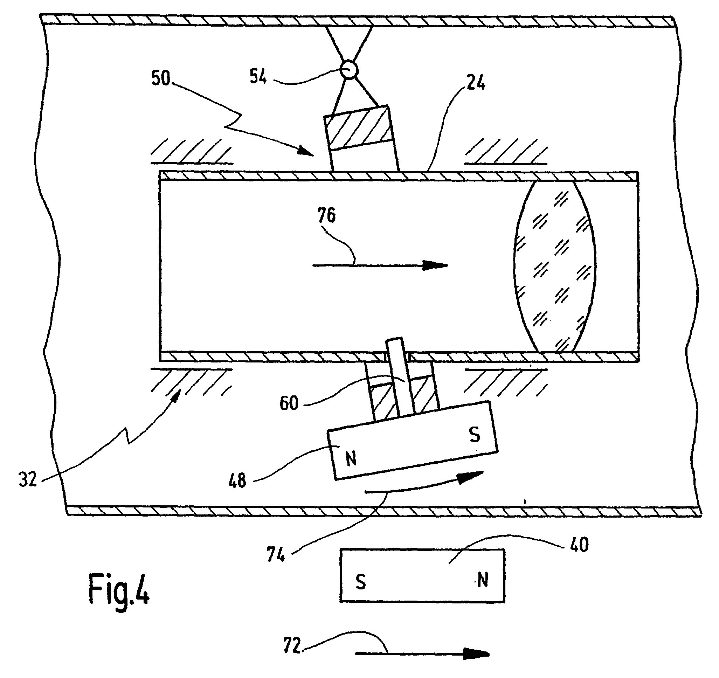

Anhand der schematisch vereinfachten Darstellung in Figuren 3 und 4 wird das Funktionsprinzip der Vorrichtung 20 näher erläutert, wobei in Figuren 3 und 4 die aus Fig. 2 übernommenen Elemente mit den gleichen Bezugszeichen versehen sind.

In Fig. 3 ist die Vorrichtung 20 in einer Betriebsstellung dargestellt, in der die Halterung 50 und damit das innere magnetisch wirksame Element 48 bezüglich ihrer Aufhängung in dem Gelenk 54 nicht ausgelenkt sind.

Die von dem äußeren magnetisch wirksamen Element 40 auf das innere Element 48 ausgeübte magnetische Anziehungskraft, die in Fig. 3 durch Pfeile 70 veranschaulicht ist, wird allein von dem Gelenk 54 aufgenommen, während das innere magnetisch wirksame Element 48 in dem Gehäuse 22 schwebend bzw. fliegend aufgehängt ist. Entsprechend ist das innere magnetisch wirksame Element 48 und auch die Halterung 50 keinerlei Gleitreibung unterworfen.

Das Bauelement 24, das mit dem inneren magnetisch wirksamen Element 48 über das Mitnehmerelement 60 in Wirkverbindung steht, das in Figuren 3 und 4 einfach als Stift dargestellt ist, der in eine Öffnung in dem Bauelement 24 eingreift, ohne mit diesem fest verbunden zu sein, d.h. ein radiales Spiel bezüglich dem Bauelement 24 aufweist, ist entsprechend in der Führung 32 im wesentlichen radial kräftefrei linear geführt. Die Anziehungskraft zwischen den Elementen 40 und 48 wirkt sich somit in keiner Weise reibungserhöhend auf die Führung des Bauelements 24 in der Führung 32 aus.

Wird das äußere magnetisch wirksame Element 40 nun, wie in Fig. 4 dargestellt ist, in Richtung eines Pfeiles 72 axial bewegt, wird das innere magnetisch wirksame Element 48 über den magnetischen Kraftschluß mit dem äußeren Element 40 um die durch das Gelenk 54 definierte Schwenkachse in Richtung eines Pfeiles 74 mit axialer Bewegungskomponente pendelartig ausgelenkt, und über das Mitnehmerelement 60 wird das Bauelement 24 in der Führung 32 entsprechend linear verschoben, wodurch das Bauelement 24 wie gewünscht positioniert bzw. lageverstellt wird. Die Positionierung des Bauelements 24 geschieht, da im wesentlichen keine Haftreibung zu überwinden ist, entsprechend ruckfrei und äußerst reibungsarm. Dadurch, daß das Bauelement 24 mit dem inneren magnetisch wirksamen Element 48 über das Mitnehmerelement 60 mit radialem Spiel verbunden ist, wirkt sich auch die radiale Bewegungskomponente des inneren magnetisch wirksamen Elements 48 nicht reibungserhöhend oder störend auf die Bewegung des Bauelements 24 in der Führung 32 aus, sondern es wird lediglich die Bewegungskomponente des Elements 48 in Richtung der Längsachse 38 entsprechend einem Pfeil 76 in Fig. 4 übertragen.

Um die Winkellage des inneren Elements 48 bei seiner Auslenkung aus der Ruhelage um die durch das Gelenk 54 definierte Schwenkachse über den Ausschwenkbereich konstant zu halten, könnte das innere Element 48 mit der Halterung 50 auch entsprechend gelenkig verbunden sein, oder die Halterung 50 könnte als gelenkiges Parallelogramm ausgebildet sein, so daß das innere Element 48 in jeder Winkellage parallel zu dem äußeren Element 40 verläuft.

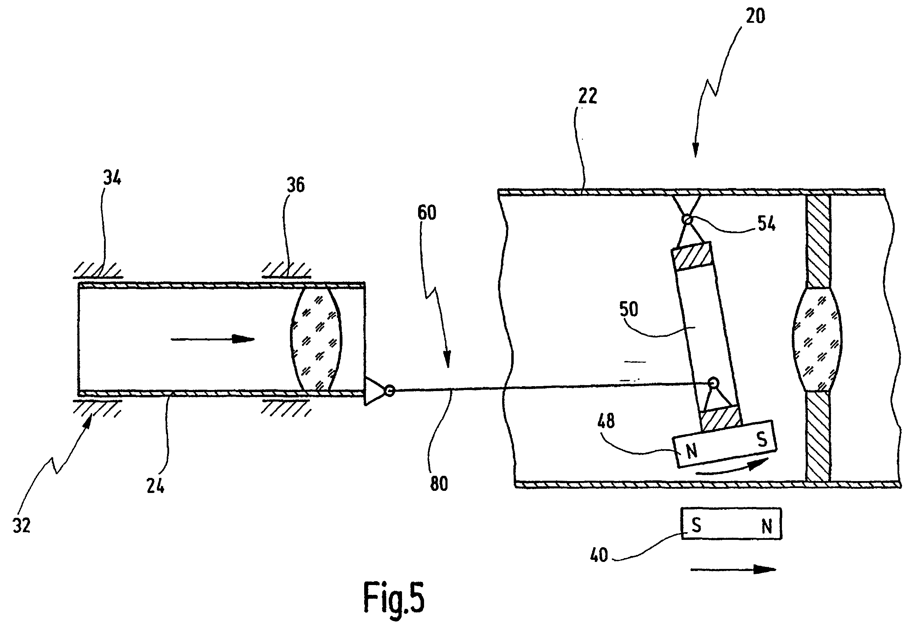

In Fig. 5 ist eine gegenüber dem vorherigen Ausführungsbeispiel gemäß Figuren 1 bis 4 geringfügig abgewandeltes Ausführungsbeispiel der Vorrichtung 20 dargestellt, bei dem das Bauelement 24 von der Halterung 50 axial beabstandet ist. Das Mitnehmerelement 60 ist in diesem Fall als längliches Zug- und Schubelement 80 ausgebildet ist, das mit der Halterung 50 einerseits und mit dem Bauelement 24 andererseits verbunden ist. Das Zug- und Schubelement 80 ist in dem gezeigten Ausführungsbeispiel sowohl mit dem Bauelement 24 als auch mit der Halterung 50 gelenkig verbunden ist, um einen Ausgleich für die radiale Bewegungskomponente des inneren magnetisch wirksamen Elements 48 bzw. der Halterung 50 bei seiner pendelartigen Verschwenkung um das Gelenk 54 zu schaffen.

Die in Fig. 5 dargestellte Ausgestaltungsform der Vorrichtung 20 eignet sich für den Fall, daß das zu positionierende Bauelement 24 distal in dem endoskopischen System angeordnet ist, während die Bedienung der Vorrichtung 20 proximal am endoskopischen System erfolgen soll.

## Patentansprüche

1. Vorrichtung zum Positionieren zumindest eines Bauelements (24) innerhalb eines endoskopischen Systems (10), mit einem hermetisch dichten Gehäuse (22), mit zumindest einem äußeren magnetisch wirksamen Element (40), das außerhalb des Gehäuses (22) angeordnet ist, und mit zumindest einem inneren magnetisch wirksamen. Element (48), das innerhalb des Gehäuses (22) angeordnet ist, wobei zwischen dem äußeren Element (40) und dem inneren Element (48) durch das Gehäuse (22) hindurch ein magnetischer Kraftschluß wirkt, wobei ferner das äußere Element (40) und das innere Element (48) bzgl. einer Längsachse (38) des Gehäuses (22) zumindest mit axialer Bewegungskomponente beweglich sind und das innere Element (48) mit dem Bauelement (24) derart in Wirkverbindung steht, daß eine Bewegung des inneren Elements (48) eine Bewegung des Bauelements (24) bewirkt, **dadurch gekennzeichnet, daß** das zumindest eine innere Element (48) in Bezug auf die Richtung der Anziehungskraft des äußeren Elements (40) über eine Halterung (50) in dem Gehäuse (22) zumindest mit axialer Bewegungskomponente hängend angeordnet ist, wobei eine dem äußeren Element (40) zugewandte Seite (68) des inneren Elements (48) frei ist, und daß das Bauelement (24) über ein Mitnehmerelement (60) mit dem inneren Element (48) derart in Wirkverbindung steht, daß das Bauelement (24) bei einer axialen Bewegung des inneren Elements (48) axial verschoben wird.

2. Vorrichtung nach Anspruch 1, **dadurch gekennzeichnet, daß** das innere Element (48) mittels der Halterung (50) um zumindest eine Schwenkachse pendelnd in dem Gehäuse (22) aufgehängt ist.

3. Vorrichtung nach Anspruch 1 oder 2, **dadurch gekennzeichnet, daß** das Bauelement (24) in einer Führung axial linear geführt ist.

4. Vorrichtung nach einem der Ansprüche 1 bis 3, **dadurch gekennzeichnet, daß** die Halterung (50) mit einem ersten Ende (52) über zumindest ein Gelenk (54) im Gehäuse (22) aufgehängt ist und am gegenüberliegenden Ende das innere Element (48) trägt.

5. Vorrichtung nach einem der Ansprüche 1 bis 4, **dadurch gekennzeichnet, daß** das innere Element (48) gelenkig mit der Halterung (50) verbunden ist, derart, daß sich die Winkellage des inneren Elements (48) in Bezug auf die Längsachse (38) des Gehäuses (22) bei der Pendelbewegung nicht ändert.

6. Vorrichtung nach einem der Ansprüche 1 bis 5, **dadurch gekennzeichnet, daß** die Halterung (50) seitlich um das Bauelement (24) verläuft.

7. Vorrichtung nach einem der Ansprüche 1 bis 6, **dadurch gekennzeichnet, daß** das innere Element (48) mit dem Bauelement (24) über das Mitnehmerelement (60) mit radialem Spiel bezüglich der Längsachse (38) des Gehäuses (22) in Wirkverbindung steht.

8. Vorrichtung nach einem der Ansprüche 1 bis 7, **dadurch gekennzeichnet, daß** das Bauelement (24) von der Halterung (50) axial beabstandet ist, und daß das Mitnehmerelement (60) als längliches Zug- und Schubelement (80) ausgebildet ist, das mit der Halterung (50) einerseits und mit dem Bauelement (24) andererseits, vorzugsweise jeweils gelenkig, verbunden ist.

9. Vorrichtung nach einem der Ansprüche 1 bis 8, **dadurch gekennzeichnet, daß** das äußere-Element (40) mit einem Bedienungselement (62) verbunden ist, das seitlich am Gehäuse (22) angeordnet ist.

10. Vorrichtung nach Anspruch 9, **dadurch gekennzeichnet, daß** das Bedienungselement (62) als Stellrad mit etwa quer zur Längsachse (38) verlaufender Drehachse ausgebildet ist, das mit dem äußeren Element (40) in Wirkverbindung steht, wobei eine Drehbewegung des Stellrades eine Bewegung des äußeren Elements (40) mit axialer Bewegungskomponente umsetzt.

## Claims

1. An apparatus for positioning at least one component (24) within an endoscopic system (10), comprising a hermetically tight housing (22), at least one external magnetically active element (40) which is arranged outside the housing (22), and at least one internal magnetically active element (48) which is arranged inside the housing (22), a magnetic force coupling acting through the housing (22) between the external element (40) and the internal element (48), it further being possible for the external element (40) and the internal element (48) to move at least with an axial movement component with reference to a longitudinal axis (38) of the housing (22), and the internal element (48) being in operational connection with the component (24) in such a way that a movement of the internal element (48) causes a movement of the component (24), **characterized in that** the at least one internal element (48) is arranged, via a holder (50) in the housing (22), hanging with reference to the direction of the attractive force of the external element (40) and at least with an axial movement component, wherein a side (68), facing the external element (40), of the internal element (48) is free, and **in that** the component (24) is in operational connection with the internal element (48) via a driver element (60) in such a way that the component (24) is axially displaced given an axial movement of the internal element (48).

2. The apparatus of claim 1, **characterized in that** the internal element (48) is suspended in pendulum fashion in the housing (22) about at least one swivel axis by means of the holder (50).

3. The apparatus of claim 1 or 2, **characterized in that** the component (24) is guided in an axially linear fashion in a guide.

4. The apparatus of anyone of claims 1 through 3, **characterized in that** the holder (50) is suspended with a first end (52) via at least one joint (54) in the housing (22), and carries the internal element (48) at the opposite end.

5. The apparatus of anyone of claims 1 through 4, **characterized in that** the internal element (48) is connected in an articulated fashion to the holder (50) in such a way that the angular position of the internal element (48) does not change with reference to the longitudinal axis (38) of the housing (22) during the pendulum movement.

6. The apparatus of anyone of claims 1 through 5, **characterized in that** the holder (50) extends on a side of the component (24).

7. The apparatus of anyone of claims 1 through 6, **characterized in that** the internal element (48) is in operational connection with the component (24) via the driver element (60) with radial play relative to the longitudinal axis (38) of the housing (22).

8. The apparatus of anyone of claims 1 through 7, **characterized in that** the component (24) is axially spaced from the holder (50), and **in that** the driver element (60) is designed as an elongated pulling and pushing element (80) which is connected, on the one hand, to the holder (50) and, on the other hand, to the component (24), preferably in an articulated fashion in each case.

9. The apparatus of anyone of claims 1 through 8, **characterized in that** the external element (40) is connected to an operating element (62) which is arranged on a side of the housing (22).

10. The apparatus of claim 9, **characterized in that** the operating element (62) is designed as an adjusting wheel which has an axis of rotation running approximately transverse to the longitudinal axis (38) and is in operational connection with the external element (40), a rotary movement of the adjusting wheel causing a movement of the external element (40) with an axial movement component.

## Revendications

1. Dispositif de positionnement d'au moins un composant (24) à l'intérieur d'un système endoscopique (10), avec un boîtier (22) hermétiquement fermé, avec au moins un élément extérieur (40) à effet magnétique qui est placé en dehors du boîtier (22), et avec au moins un élément intérieur (48) à effet magnétique qui est placé dans le boîtier (22), une adhérence magnétique étant présente entre l'élément extérieur (40) et l'élément intérieur (48) à travers le boîtier (22), l'élément extérieur (40) et l'élément intérieur (48) étant de plus mobiles par rapport à un axe longitudinal (38) du boîtier (22) au moins avec une composante de déplacement axiale et l'élément intérieur (48) étant en liaison active avec le composant (24) de telle manière qu'un mouvement de l'élément intérieur (48) provoque un mouvement du composant (24), **caractérisé en ce qu'**au moins un élément intérieur (48) est placé suspendu dans le boîtier (22) par l'intermédiaire d'un support (50) avec au moins une composante de déplacement axiale par rapport à la direction de la force d'attraction de l'élément extérieur (40), une face (68) de l'élément intérieur (48) tournée vers l'élément extérieur (40) étant libre, et **en ce que** le composant (24) est en liaison active avec l'élément intérieur (48) par l'intermédiaire d'un élément d'entraînement (60) de telle manière que le composant (24) est déplacé axialement en cas de mouvement axial de l'élément intérieur (48).

2. Dispositif selon la revendication 1, **caractérisé en ce que** l'élément intérieur (48) est suspendu dans le boîtier (22) de manière oscillante autour d'au moins un axe de pivotement au moyen du support (50).

3. Dispositif selon la revendication 1 ou 2, **caractérisé en ce que** le composant (24) est guidé axialement de manière rectiligne dans un guidage.

4. Dispositif selon l'une des revendications 1 à 3, **caractérisé en ce que** le support (50) est suspendu avec une première extrémité (52) par l'intermédiaire d'au moins une articulation (54) dans le boîtier (22) et porte l'élément intérieur (48) à l'extrémité opposée.

5. Dispositif selon l'une des revendications 1 à 4, **caractérisé en ce que** l'élément intérieur (48) est relié de façon articulée au support (50), de telle manière que la position angulaire de l'élément intérieur (48) par rapport à l'axe longitudinal (38) du boîtier (22) ne varie pas lors du mouvement oscillatoire.

6. Dispositif selon l'une des revendications 1 à 5, **caractérisé en ce que** le support (50) s'étend latéralement autour du composant (24).

7. Dispositif selon l'une des revendications 1 à 6, **caractérisé en ce que** l'élément intérieur (48) est en liaison active avec le composant (24) par l'intermédiaire de l'élément d'entraînement (60) avec un jeu radial par rapport à l'axe longitudinal (38) du boîtier (22).

8. Dispositif selon l'une des revendications 1 à 7, **caractérisé en ce que** le composant (24) est à distance axiale du support (50) et **en ce que** l'élément d'entraînement (60) est conformé en élément de traction et de poussée allongé (80) qui est relié d'une part au support (40) et d'autre part au composant (24), de préférence à chaque fois de façon articulée.

9. Dispositif selon l'une des revendications 1 à 8, **caractérisé en ce que** l'élément extérieur (40) est relié à un élément de commande (62) qui est placé latéralement sur le boîtier (22).

10. Dispositif selon la revendication 9, **caractérisé en ce que** l'élément de commande (62) est conformé en roue de réglage à axe de rotation approximativement transversal à l'axe longitudinal (38), qui est en liaison active avec l'élément extérieur (40), un mouvement de rotation de la roue de réglage provoquant un mouvement de l'élément extérieur (40) avec une composante axiale de déplacement.
